# EUROPÄISCHE PATENTSCHRIFT

(11) **EP 0 963 213 B1**
(45) Veröffentlichungstag und Bekanntmachung des Hinweises auf die Patenterteilung: **15.01.2003**
(21) Anmeldenummer: 98909327.3
(22) Anmeldetag: 04.02.1998
(51) Int. Cl.: A61M 5/32, A61M 5/50

(54) **UNIVERSALSICHERHEITSSPRITZE**
UNIVERSAL SAFETY SYRINGE
SERINGUE DE SECURITE UNIVERSELLE

(30) Priorität: 15.02.1997 DE 19705892
(43) Veröffentlichungstag der Anmeldung: 15.12.1999
(73) Patentinhaber: Siekmann GmbH, 24536 Neumünster (DE)
(72) Erfinder: SIEKMANN, Manfred, D-24536 Neumünster (DE)
(74) Vertreter: Biehl, Christian, Dipl.-Phys.
(86) Internationale Anmeldenummer: DE9800304
(87) Internationale Veröffentlichungsnummer: WO98035713

(56) Entgegenhaltungen:
- EP-A- 0 646 384
- DE-A- 4 340 082
- US-A- 5 344 403
- US-A- 5 520 649

## Beschreibung

Die Erfindung betrifft eine Universalsicherheitsspritze nach dem Oberbegriff des Hauptanspruches.

Bei einer Anzahl Sicherheitsspritzen werden, wie beispielsweise in der Deutschen Patentschrift 43 40 082 gezeigt, die Injektionskanülen nach Gebrauch in den Zylinder zurückbefördert, um dort sicher verwahrt ohne Verletzungsgefahr beim weiteren Hantieren mit der Spritze dauernd zu verbleiben. Allerdings wird meist wie in o.g. Druckschrift auch ein neues Material, hier beipielsweise ein elastomerer Werkstoff vorgeschlagen, oder es können die üblichen Nadeln mit Luer-Konus nicht weiter verwendet werden.

In weiteren Ausführungsformen, wie etwa der in dem US-Patent 5 336 198 beschriebenen Ausführung werden sogar gesonderte Mittel vorgeschlagen, um die Kanüle im Zylinder seitlich zu verkippen, damit sie nicht wieder herausgeschoben werden kann. All diesen Vorschlägen ist leider eine nicht unerhebliche Komplexität gemein, die diese Sicherheitsspritzen teuer und nur für spezielle Anwendungen geeignet erscheinen läßt.

Leider ist jedoch in der jetzigen Medizinpraxis das Bedürfnis nach Sicherheitsspritzen derart groß, daß man Sicherheitsspritzen eigentlich immer einsetzen möchte. Bisher ist dies aufgrund der komplexen Geometrien, mit denen die Kanülen und/oder Spritzen zu versehen waren, nicht möglich gewesen.

Um die Sicherheitsspritzen mit einziehbarer Kanüle jedoch generell einführen zu können, muß als Vorbedingung eine sog. "Abwärts"-Kompatibilität zu bisher verwandtem Material gegeben sein, so daß auch die bisher schon in großer Vielfalt vorhandenen Kanülen aufgesteckt werden können. Ansonsten ergäbe sich als Vorbedingung, daß sofort für alle denkbaren Zwecke und an allen Einsatzorten neuartige Kanülen in allen Größen vorhanden sein müssen, um zu vermeiden, daß für eine Übergangszeit beide Systeme nebeneinander - mit allen Möglichkeiten der Verwechslung, Problemen der doppelten Vorratshaltung und gleichzeitiger unterschiedlicher Handhabungen existieren würden.

Da aber bisher bekannte Sicherheitsspritzen jeweils ihre eigenen Kanülen bzw. Kanülensockel benötigen, die häufig aber nicht in der gewünschten Vielfalt angeboten bzw. vorgehalten werden konnten, ist es unbedingt Vorbedingung, die bisherigen Kanülen weiterverwenden zu können.

Wenn jedoch eine Universalsicherheitsspritze mit einziehbarer Kanüle benutzt wird, kann durch unachtsames Hantieren mit dem Kolben dieser im Zylinder nach vorn geschoben werden, wo er schon vor Benutzung mit dem Kanülensockel in Verbindung tritt. Dies kann beispielsweise bei der Aufdrückbewegung der Kanüle geschehen. Selbstverständlich muß dies nun aber unbedingt vermieden werden, weil dann - gerade bei Notsituationen vielleicht zunächst unbemerkt - beim weiteren Aufziehen der Spritze der Kanülensockel schon (zu früh) in den Zylinder zurückgezogen wird.

Bei bisher bekannten Sicherheitsspritzen, wie beispielsweise derjenigen aus der DE 38 44 150, wurde dies durch Sicherheitsorgane vermieden, die vor dem Injizieren entfernt wurden, um den Kolben ganz nach innen drücken zu können. Separat vorgesehene Sicherheitsorgane jedoch sind bei einem universellen Einsatz höchst unerwünscht, da sie die Spritzen nicht nur verteuern, sondern auch im bisher üblichen Normalgebrauch die Handhabung der Spritzen verkomplizieren.

Erfindungsgemäß werden die Nachteile des Standes der Technik nun durch eine Universalsicherheitsspritze mit den Merkmalen des Hauptanspruches gelöst. Die Unteransprüche geben vorteilhafte Ausführungsformen der Erfindung wieder.

Insbesondere ist vorteilhaft, daß die Sicherheitsspritze im Vergleich zu konventionellen Spritzen kein zusätzliches Teil besitzt und damit nicht nur die wirtschaftlichste Lösung einer Sicherheitsspritze darstellt, sondern auch die Bewegungsabläufe bei der Handhabung genau die gleichen wie bei der konventionellen Spritze sind. Durch den vorhandenen äußeren Luer-Standard-Konus können alle konventionellen Kanülen aufgesetzt werden. Es können aber auch, da das Prinzip keine vormontierte Kanüle erfordert, passende (Sicherheits)-Kanülen auf ihren Kanülensockeln erst kurz vor der Anwendung gewählt und eingesetzt werden.

Damit entspricht die beschriebene Sicherheitsspritze sowohl der DIN 13098 als auch der ISO 7886. Insbesondere ist vorteilhaft, daß in einer bevorzugten Ausführungsform ein vorzeitiges Herunterdrücken, das die Spritze unbrauchbar machen könnte, nicht möglich ist.

Bei exzentrischer Konusanordnung kann durch eine Führungsrille oder, wie vorgeschlagen zwei gegenüberliegende Führungsrillen eine Möglichkeit geschaffen werden, auch für diesen Fall das erfindungsgemäße Prinzip einzusetzen, das sich im übrigen außer für alle Spritzengrößen auch für zweiteilige und dreiteilige Spritzen mit einer zusätzlichen elastischen Kolbendichtung am Kolbenende eignet.

Die erfindungsgemäße Universalsicherheitsspritze zeichnet sich durch einen Kanülensockel aus, der in der Schutzkappe fest gegen einen Anschlag eingeschoben ist, weiter eine Schutzkappe, die auf einen einem Luer-Konus entsprechenden Konus des Zylinders außen aufschiebbar ist, wobei an ihrer Innenseite vor einer zur Aufnahme des Kanülensockels ausgebildeten Ausnehmung eine den Aufschubweg begrenzende Verengung vorgesehen ist.

Die den Kanülensockel aufnehmende Ausnehmung mit einer Hinterschneidung gegen ein Entfernen des Kanülensockels nach vorn versehen, und mit einer durch ein Aufweiten des Konus von innen aufhebbaren Hinterschneidung, die zur Sicherung des Kanülensockels gegen ein unbeabsichtigtes Hineinschieben während des Einstechens vorgesehen ist, wobei diese letztere Hinterschneidung von einem zylindrischen Ende des Kolben beim letzten Stück des Vorschubs aufgeweitet wird, während ein bevorzugt pilzförmiger Kolbenkopf in einen im wesentlichen zylindrischen Freiraum mit hinter den pilzförmigen Kopf greifenden Randelemente eingedrückt wird.

Eine weitere vorteilhafte Ausgestaltung zeichnet sich durch an dem Kolbenbetätigungsende vorgesehene, nach außen vorgespannte Lappen aus, die durch entsprechende Lappenstützen am Kolben vor Befüllung in einer im wesentlichen längs der Spritzenachse ausgerichteten Stellung gehalten sind, um so ein Eindrücken des Kolbens in den Zylinder zu verhindern.

Diese Lappen werden z. B. als zwei einander im wesentlichen gegenüberliegende, mit Filmscharnieren am Betätigungsende des Kolbens befestigte, zum Zylinder weisende Spreizlappen ausgeführt, die in einer an der Einbringmündung für den Kolben vorgesehenen Ausnehmung in der Zylindermündung zur Kolbeneinführung zunächst klemmgelagert sind.

Auch bei einer exzentrischen Anordnung des Konus und des den Kanülenhalter greifenden Kolbenkopfes kann die Erfindung eingesetzt werden, indem der Kolben zusätzlich mit vorspringenden Kanten in entsprechenden Führungsrillen an der Innenseite des Kolbens geführt ist.

Weitere Merkmale und Vorteile der Erfindung ergeben sich aus nachfolgender Beschreibung eines bevorzugten Ausführungsbeispiels anhand der beigefügten Zeichnung.

Dabei zeigt:
- Fig. 1: die erfindungsgemäße Verbindung des Kanülen-Sockels in der Spitze des Zylinders bei aufgeschobener Schutzkappe und nicht eingeschobenem Kolbenkopf in einer zweiteiligen Spritze,
- Fig. 2: eine der Fig. 1 entsprechende Darstellung mit eingeschobenem Kolbenkopf ohne Schutzkappe,
- Fig. 3: eine der Fig. 1 entsprechende Darstellung einer dreiteiligen Spritze,
- Fig. 4: eine der Fig. 2 entsprechende Darstellung mit einer dreiteiligen Spritze,
- Fig. 5: die erfindungsgemäße Spritze in einem weiteren bevorzugten Ausführungsbeispiel mit noch im Zylinder gelagerten Klemmlappen,
- Fig. 6: die Darstellung der Fig. 5 mit hereingeschobenem Kolben und nach außen verlagerten Klemmlappen, und
- Fig. 7: die Schlußposition mit wieder herausgezogenem Kolben,
- Fig. 8: eine Darstellung einer exzentrischen Konusanordnung, und
- Fig. 9: einen Querschnitt durch den Kolben, bei dem die Führungsrillen erkennbar sind.

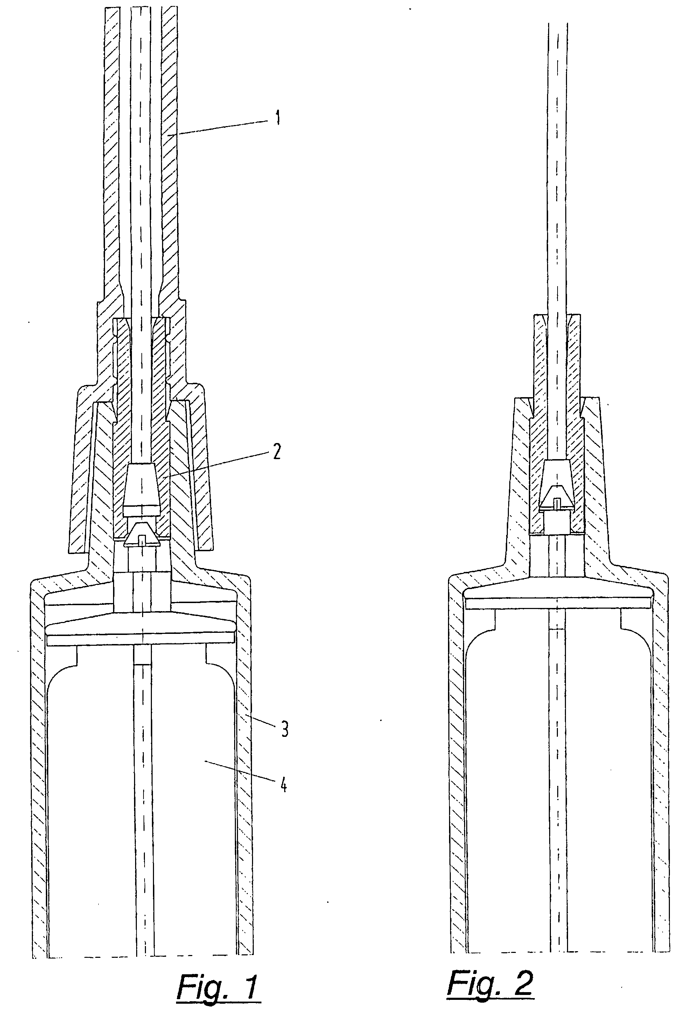

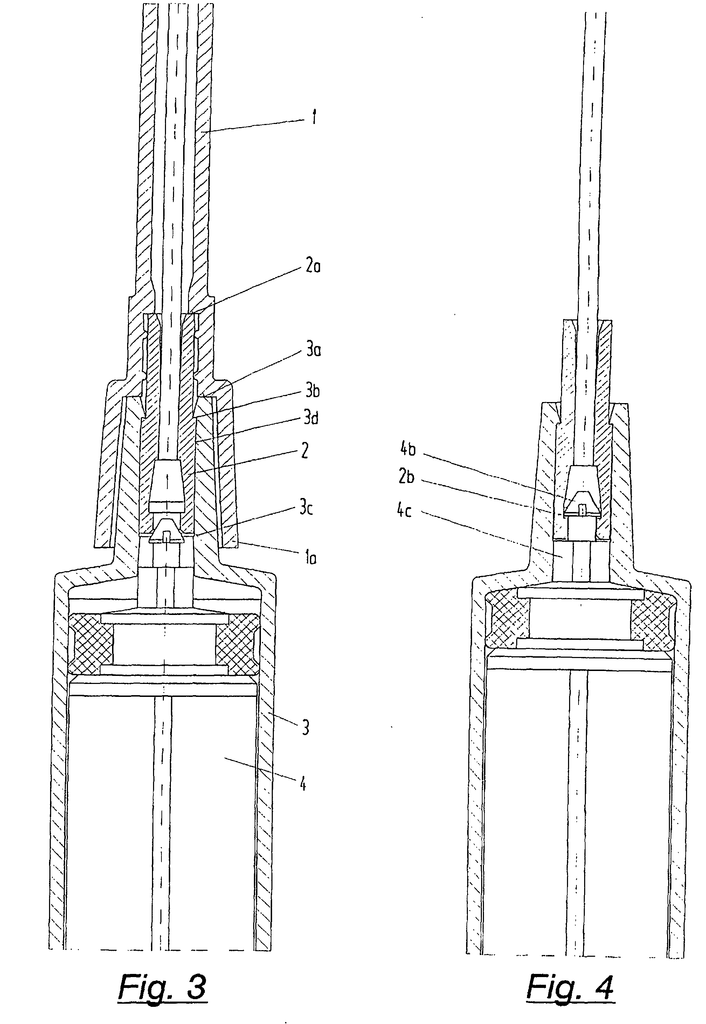

Die in der Fig. 1 dargestellte Spritze ist mit einer Schutzkappe 1 versehen, die auf einem Kanülensockel 2 aufklemmt und mit einem Bund 1a (Fig. 3) einen Konus am Zylinder anpaßt, der dem Luer-Standard entspricht. Dieser Bund 1a dient gleichzeitig als Führung beim Einführen des Kanülensockels 2, wenn dieser in den Zylinder 3 eingeführt wird. Er schützt den Kanülensockel gegen Beschädigung und Verunreinigungen vor der Einführung.

Der Kanülensockel 2 ist mit einem Anschlag 2a versehen, der gegen einen entsprechenden Anschlag der Schutzkappe 1 beim Aufsetzen der Kanüle für die Universalsicherheitsspritze stößt. Die Schutzkappe 1 dient dadurch zusätzlich als Anschlag beim Einführen des Kanülensockels in den Zylinder, indem durch Anstoßen der Schutzkappe auf den Zylinder an einem weiteren Anschlag 3a die Position des Kanülensockels 2 im Zylinder 3 bestimmt wird.

Vorteilhafterweise ist der Zylinder 3 mit einem Hinterschnitt 3b versehen, der bei der vorgewählten Einschubtiefe hinter eine entsprechende Durchmesserverringerung des Kanülensockels 2 eingreift und so eine einmal eingesteckte Kanüle gegen ein Abziehen nach vorn sichert. Andererseits ist aber auch ein weiterer Hinterschnitt 3c als Sockel in entgegengesetzter Richtung vorgesehen, der beim Einstechen der Kanüle ein weiteres Einschieben des Sockels verhindert. Dieser Sockel ist jedoch nicht so breit wie der obig genannte Sockel, sondern gerade derart dimensioniert, daß bei einem Einschieben eines den Zylinder an diesem Ort ein wenig aufweitenden Kolbenendstückes es dem Kanülensockel 2 ermöglicht wird, nun durch die aufgeweitete Öffnung nach innen in den Kolben gezogen zu werden.

Zwischen Kanülensockel und Zylinder können zusätzlich eine oder eine Mehrzahl von Dichtlippen 3d vorgesehen sein, die an diesem Ort den Sockel abdichten.

In der Fig. 4 ist mit Bezugszeichen 4c der zylinderische Teil des Kolben bezeichnet, der den Zylinder unter dem Sitz des Kanülensockels aufspreizt, um den Hinterschnitt 3c unwirksam werden zu lassen, wenn nach einer Abgabe eines Medikaments die Spritze unbrauchbar gemacht werden soll, indem die Kanüle eingezogen wird. Zwischen der in dem Kanülensockel vorgesehenen Hinterschneidung 2b und dem Kolbenkopf, der hier eingeführt wird, wird ein geringes Spiel gelassen, das jedoch ausreicht, daß die Kanüle durch Gravitation im zurückgezogenen Zustand zur Seite fällt und so nicht mehr durch die gleiche Öffnung herausgeschoben werden kann.

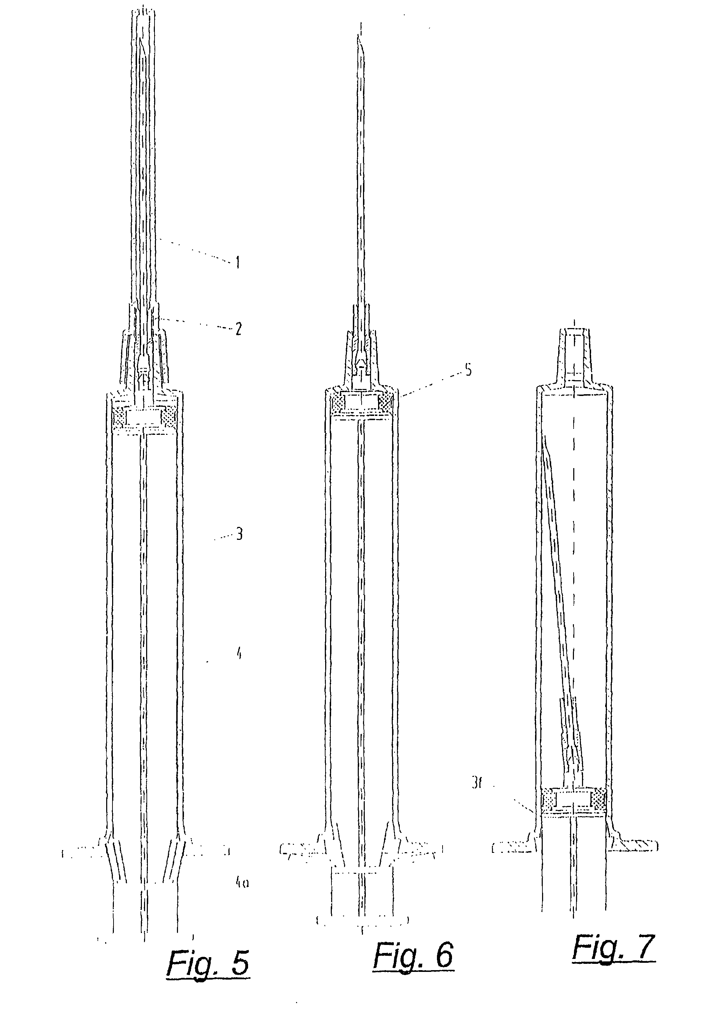

In einer bevorzugten Ausführungsform der Erfindung, die in der Fig. 5 und 6 dargestellt ist, werden zusätzlich zwei mit einem Filmscharnier an dem Kolbenbetätigungsende vorgesehene Lappen 4a vorgeschlagen, die in einer entsprechenden, beispielsweise ringförmigen Ausnehmung am offenen Griffplattenende des Zylinders eingerastet sind. Solange der Kolben 4 nicht herausbewegt wird, werden sie durch ihre Filmscharniervorspannung nach außen in der ringförmigen Ausnehmung gespannt verbleiben. Falls jedoch der Kolben zurückgezogen wird, werden sie weiter nach außen sicher herauslagern, so daß bei erneutem Eindrücken des Kolbens sie nicht wieder in die Ausnehmung zurückkehren, sondern flach auf die Griffplatte außen an dem Zylinder aufliegen. Der Kolben kann dadurch bis zum Boden des Zylinders eingedrückt werden um in den Kanülensockel mit dem Kolbenkopf einzudringen und diesen bei einer anschließenden Rückbewegung mitzunehmen. Vor einer ersten Rückbewegung des Kolbens können jedoch fast beliebig hohe Kräfte auf den Kolben ausgeübt werden, er wird sich nicht in den Kanülenstock hineinbewegen.

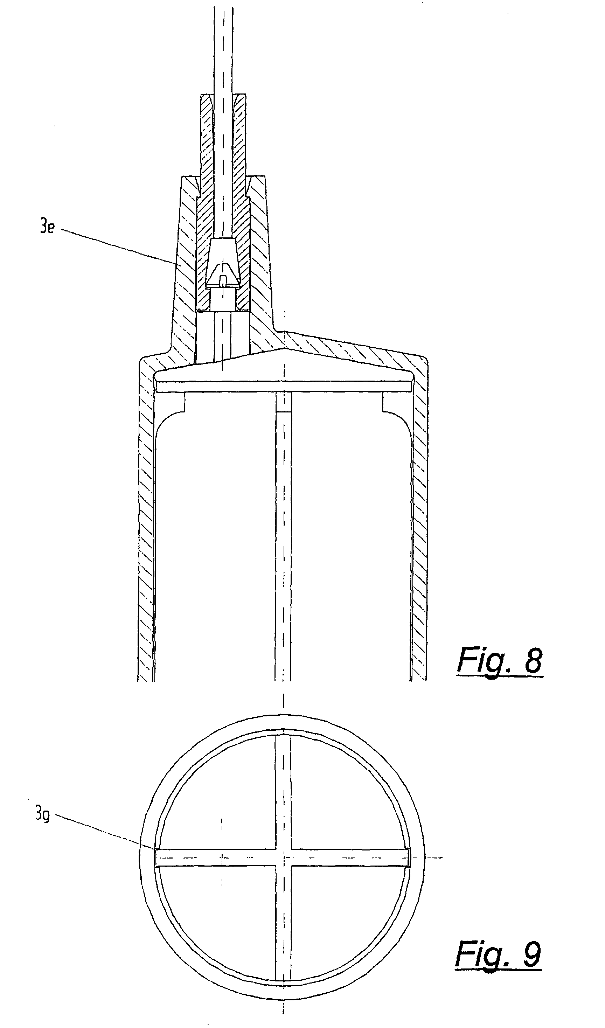

Schließlich ist noch in der Fig. 8 ein exzentrischer Konus 3e dargestellt, der jedoch ohne Probleme auf die gleiche Weise mit einem Kanülensockel versehen werden kann, wenn der Kolben beispielsweise durch zwei vorspringende Kanten, die in entsprechenden Führungsrillen 3e auf der Innenseite des Kolbens geführt werden, verdrehsicher ausgeführt wird.

Schließlich bleibt noch darauf hinzuweisen, daß sich die Ausführungsformen der Fig. 1 und 2 gegenüber denen der Fig. 3 und 4 lediglich durch einen zusätzlichen elastischen Dichtring unterscheiden, der außen um einen entsprechenden Dichtringträger am Kolbenende aufgesetzt ist.

## Patentansprüche

1. Universalsicherheitsspritze mit einer Schutzkappe (1), einem Zylinder (3), einem Kolben (4), einem Kanülensockel (2) und einer auf dem Kanülensockel (2) gelagerten Kanüle,
wobei eine den Kanülensockel (2) aufnehmende Ausnehmung mit einer ersten Hinterschneidung (3b) gegen ein Abziehen des Kanülensockels versehen ist, und mit einer zweiten Hinterschneidung (3c), die zur Sicherung des Kanülensockels (2) gegen ein unbeabsichtigtes Hineinschieben in Zylinder während des Einstechens vorgesehen ist,
der Kolben (4) mit einem pilzförmigen, mit dem Kanülensockel (2) in Zugkontakt tretenden Kolbenkopf (4b) ausgebildet ist,
**dadurch gekennzeichnet, daß**
- der Kanülensockel (2) in der Schutzkappe (1) fest gegen einen Anschlag gelagert einschiebbar ausgebildet ist,
- ein Konus am Zylinder (3) ausgebildet ist, auf den die Schutzkappe (1) außen aufschiebbar ist, der in seiner Form einem Luer-Konus entspricht,
- die Schutzkappe (1) an ihrer Innenseite mit einer den Aufschubweg des Kanülensockels (2) im Konus des Zylinders begrenzenden Verengung vor einer zur Aufnahme des Kanülensockels (2) ausgebildeten Ausnehmung versehen ist,
- wobei die zweite Hinterschneidung (3c), die gegen ein Hineinschieben in den Konus des Zylinders vorgesehen ist, durch ein Aufweiten des Konus von innen aufhebbar ausgebildet ist,
- der Kolben (4) mit einem den Konus zylindrisch aufweitenden Abschnitt ausgebildet ist.

2. Universalsicherheitsspritze nach Anspruch 1, **dadurch gekennzeichnet, daß** der Kolbenkopf (4b) kegelig ausgebildet ist, während der Kanülensockel (2) eine unter Belassung eines im wesentlichen zylindrischen Freiraumes gebildete Buchse mit hinter den pilzförmigen Kopf greifendem Rand aufweist.

3. Universalsicherheitsspritze nach einem der vorangehenden Ansprüche, **gekennzeichnet durch** an dem Kolbenbetätigungsende vorgesehene, nach außen vorgespannte Lappen (4a), die **durch** entsprechende Lappenstützen am Kolben vor Befüllung in einer im wesentlichen längs der Spritzenachse ausgerichteten Stellung gehalten sind.

4. Universalsicherheitsspritze nach Anspruch 3, **gekennzeichnet durch** zwei einander im wesentlichen gegenüberliegende, mit Filmscharnieren am Betätigungsende des Kolbens befestigte, zum Zylinder weisende Spreizlappen (4a), die in einer an der Einbringmündung für den Kolben (4) vorgesehenen Ausnehmung im Zylinder (3) zunächst klemmgelagert sind.

5. Universalsicherheitsspritze nach einem der vorangehenden Ansprüche, **gekennzeichnet durch** eine exzentrische Anordnung des Konus und des den Kanülenhalter (2) greifenden Kolbenkopfes (4b), bei der der Kolben (4) zusätzlich mit vorspringenden Kanten in entsprechenden Führungsrillen (3g) an der Innenseite des zylinders geführt ist.

## Claims

1. Universal safety syringe having a protective cap (1), a cylinder (3), a plunger (4), a cannula base (2) and a cannula mounted on the cannula base (2), it being the case that a recess which receives the cannula base (2) is provided with a first undercut (3b) which prevents the cannula base from being pulled out, and with a second undercut (3c) which is provided for safeguarding the cannula base (2) against being pushed into the cylinder unintentionally during insertion of the syringe,
the plunger (4) is designed with a mushroom-shaped plunger head (4b), which comes into pulling contact with the cannula base (2),
**characterized in that**
- the cannula base (2) is designed such that it can be pushed firmly against a stop in the protective cap (1),
- a cone is formed on the cylinder (3), onto which the protective cap (1) can be pushed externally and which corresponds, in terms of shape, to a Luer cone,
- the protective cap (1) is provided, on its inside, with a narrowed section, which delimits the push-on travel of the cannula base (2) in the cone, in front of a recess designed for receiving the cannula base (2),
- it being the case that the second undercut (3c), which is provided for safeguarding the cannula base against being pushed into the cone of the cylinder, is designed such that its effect can be overcome by the cone being widened from the inside,
- the plunger (4) is designed with a section which widens the cone cylindrically.

2. Universal safety syringe according to Claim 1, **characterized in that** the plunger head (4b) is of conical design, while the cannula base (2) has a bushing, which is formed with an essentially cylindrical cavity, with a border engaging behind the mushroom-shaped head.

3. Universal safety syringe according to one of the preceding claims, **characterized by** lugs (4a) which are provided at the actuating end of the plunger, are prestressed outwards and, prior to the filling operation, are retained, by corresponding lug supports on the plunger, in a position in which they are aligned essentially along the syringe axis.

4. Universal safety syringe according to Claim 3, **characterized by** two spreader lugs (4a) which are located essentially opposite one another, are fastened at the actuating end of the plunger by film hinges, are oriented towards the cylinder and are initially clamped in a recess, provided at the introduction opening for the plunger (4), in the cylinder (3).

5. Universal safety syringe according to one of the preceding claims, **characterized by** an eccentric arrangement of the cone and of the plunger head (4b) which grips the cannula holder (2), in the case of which the plunger (4) is additionally guided by way of projecting edges in corresponding guide grooves (3g) on the inside of the cylinder.

## Revendications

1. Seringue de sécurité universelle comprenant un capuchon de protection (1), un cylindre (3), un piston (4) un socle de canule (2), et une canule montée sur le dit socle (2),
une cavité, destinée à recevoir le socle de canule (2), étant pourvue d'une première contre-dépouille (3b), qui empêche le retrait du socle de canule (2), et d'une deuxième contre-dépouille (3c), qui empêche un enfoncement accidentel du socle de canule (2) dans le cylindre, pendant l'insertion,
le piston (4) présentant une tête (4b) en forme de champignon, qui entre en contact de traction avec le socle de canule (2),
**caractérisée en ce que**
- le socle de canule (2) est réalisé de manière à pouvoir être inséré dans le capuchon de protection (1) pour prendre appui ferme contre une butée,
- le cylindre (3) présente un cône sur lequel le capuchon de protection (1) peut être extérieurement glissé, la forme du dit cône correspondant à celle d'un cône Luer,
- le capuchon de protection (1) présente, sur sa surface interne, un étranglement qui limite la course du socle de canule (2), dans le cône du cylindre, en amont d'une cavité qui accueille le dit socle de canule (2),
- la deuxième contre-dépouille (3c), qui est prévue pour empêcher un enfoncement dans le cône du cylindre, étant réalisée de manière à pouvoir être retirée de l'intérieur par un élargissement du cône,
- le piston (4) étant formé avec une section qui dilate cylindriquement le cône.

2. Seringue de sécurité universelle selon la revendication 1, **caractérisée en ce que** la tête de piston (4b) est réalisée conique, tandis que le socle de canule (2) présente un manchon qui est formé de manière à laisser un espace libre, essentiellement cylindrique, avec un bord de prise derrière la tête en forme de champignon.

3. Seringue de sécurité universelle selon l'une des revendications précédentes, **caractérisée par** des pattes (4a) prévues à l'extrémité d'actionnement du piston et précontraintes vers l'extérieur, qui sont maintenues, avant le remplissage, par des éléments d'appui adéquats, dans une position orientée essentiellement selon l'axe de la seringue.

4. Seringue de sécurité universelle selon la revendication 3, **caractérisée par** deux pattes à expansion (4a), essentiellement opposées l'une à l'autre, fixées par des articulations minces à l'extrémité d'actionnement du piston, saillant en direction du cylindre, qui sont ensuite bloquées dans une cavité prévue dans le cylindre (3), à l'orifice d'entrée pour le piston (4).

5. Seringue de sécurité universelle selon l'une des revendications précédentes, **caractérisée par** une disposition excentrique du cône et de la tête (4b) du piston, retenant le socle de canule (2), le piston (4) étant en outre guidé par des nervures en saillies, dans des gorges de guidage (3g) correspondantes à la surface intérieure du dit cylindre.
